(19) Europäisches Patentamt
European Patent Office
Office européen des brevets

(11) Numéro de publication: **0 022 408**
**A1**

(12) **DEMANDE DE BREVET EUROPEEN**

(21) Numéro de dépôt: 80401004.9

(22) Date de dépôt: 02.07.80

(51) Int. Cl.$^3$: **C 07 D 207/08**
//C07C103/38

(30) Priorité: 06.07.79 FR 7917582

(43) Date de publication de la demande: 14.01.81 Bulletin 81/2

(84) Etats Contractants Désignés: FR

(71) Demandeur: ROUSSEL-UCLAF
35, boulevard des Invalides
F-75007 Paris(FR)

(72) Inventeur: Clemence, François
2, rue Turgot
F-75009-Paris(FR)

(72) Inventeur: Le Martret, Odile
42, avenue de Versailles
F-75016 Paris(FR)

(72) Inventeur: Fournex, Robert
8, rue du Commandant Rivière
F-75008 Paris(FR)

(74) Mandataire: Bourgouin, André et al,
ROUSSEL-UCLAF Boîte postale no 9
102, route de Noisy F-93230 Romainville(FR)

(54) **Nouveaux dérivés de l'alpha-phényl 2-pyrrolidineméthanol et leurs sels, procédé de préparation, application à titre de médicaments et compositions les renfermant.**

(57) L'invention concerne les dérivés de l'$\alpha$-phényl 2-pyrrolidine méthanol et leurs sels avec les acides, de formule I :

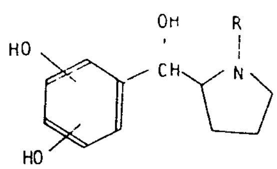

(I)

où R est hydrogène ou alcoyle de 1 à 5 carbones, sous toutes les formes stéréoisomères isolées ou en mélange, racémiques ou optiquement actives ainsi que leur préparation, leur application comme médicaments, notamment bronchodilatateurs, hypotenseurs et antidépresseurs et les compositions pharmaceutiques les renfermant.

EP 0 022 408 A1

Nouveaux dérivés de l'α-phényl 2-pyrrolidineméthanol et leurs sels, procédé de préparation, application à titre de médicaments et compositions les renfermant.

La présente invention concerne de nouveaux dérivés de l'α-phényl 2-pyrrolidineméthanol ainsi que leurs sels, le procédé de préparation, l'application à titre de médicaments de ces nouveaux dérivés et les compositions les renfermant.

L'invention a pour objet de nouveaux dérivés de l'α-phényl 2-pyrrolidineméthanol, ainsi que leurs sels d'addition avec les acides minéraux ou organiques, caractérisés en ce qu'ils répondent à la formule générale (I)

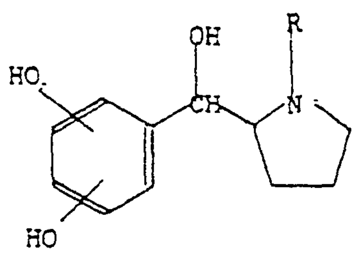

(I)

dans laquelle R représente un atome d'hydrogène ou un radical alcoyle renfermant de 1 à 5 atomes de carbone, sous toutes leurs formes stéréoisomères isolées ou en mélange, racémiques ou optiquement actives.

Dans la formule générale (I) et dans ce qui suit, le terme radical alcoyle renfermant de 1 à 5 atomes de carbone

BAD ORIGINAL

désigne, par exemple, un radical méthyl, éthyl, propyl, isopropyl, butyl ou pentyl.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, propionique, benzoïque, maléique, fumarique, succinique, tartrique, citrique, oxalique, glyoxylique, aspartique, alcanesulfoniques, tels que les acides méthane ou éthanesulfoniques, arylsulfoniques, tels que les acides benzène, ou paratoluène sulfoniques et arylcarboxyliques.

Parmi les produits, objet de l'invention, on peut citer notamment les dérivés répondant à la formule (I) ci-dessus, ainsi que leurs sels d'addition avec les acides minéraux ou organiques, caractérisés en ce que, dans ladite formule (I), les groupements hydroxyles sont en position 3 et 4 ou 3 et 5, et R représente un atome d'hydrogène.

Parmi ceux-ci, on peut citer plus particulièrement le 4-/α-hydroxy (pyrrolidin-2-yl) méthyl/ 1,2-benzène diol sous ses formes optiquement actives et racémique, ainsi que ses sels, et tout particulièrement l'oxalate acide de l'isomère A du 4-/α-hydroxy (pyrrolidin-2-yl) Méthyl/ 1,2-benzène diol sous ses formes optiquement actives ou racémique.

Les dérivés de formule (I) ci-dessus, possèdent deux carbones asymétriques, et peuvent donc exister sous différentes formes isomères. La présente invention a notamment pour objet ces différentes formes.

Ces différentes formes peuvent être obtenues séparément par des méthodes connues en soi. Les racémates diastéréoisomères seront dans ce qui suit, dénommés par les termes isomère A et isomère B; ils peuvent être obtenus séparément par des méthodes connues, par exemple par cristallisation sélective, par chromatographie, ou par des préparations stéréosélectives. On nomme par convention isomère A, l'isomère dont la constante de couplage des atomes d'hydrogène portés par les carbones asymétriques est la plus forte, et isomère B celui dont la constante de couplage est la plus faible.

Des exemples de préparation stéréosélective et de séparation par chromatographie sont donnés ci-après dans la partie expérimentale.

Ces racémates A et B peuvent être résolus en leurs énantiomères optiquement actifs par des méthodes également connues, telles que, par exemple, la formation de sels au moyen d'acides optiquement actifs.

Bien entendu, les mélanges des différents isomères des dérivés de formule (I), et en particulier les mélanges de racémates diastéréoisomères desdits dérivés, entrent dans le cadre de l'invention.

L'invention a également pour objet un procédé de préparation des dérivés, tels que définis par la formule (I) ci-dessus, ainsi que de leurs sels, caractérisé en ce que l'on fait réagir le bromure de 4-bromobutyl triphénylphosphonium avec un aldéhyde de formule (II)

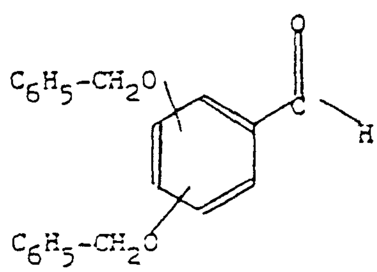

(II)

pour obtenir un produit de formule (III):

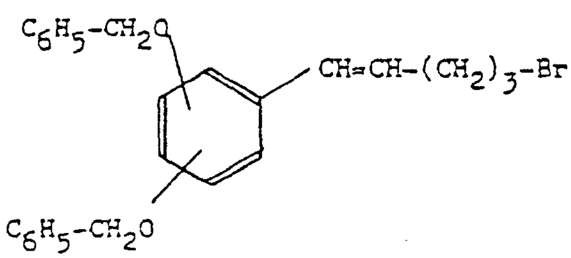

(III)

que l'on fait réagir avec un amine de formule (IV):

$R'-NH_2$ (IV)

dans laquelle R' représente un radical benzyle ou un radical alcoyle renfermant de 1 à 5 atomes de carbone, pour obtenir un produit de formule (V):

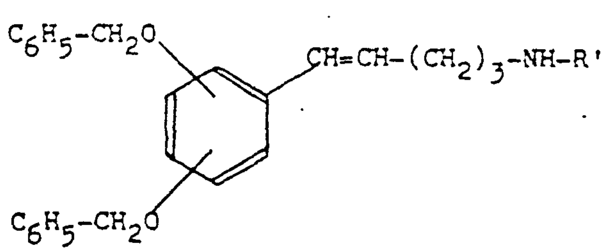

(V)

BAD ORIGINAL

dans laquelle R' a la signification déjà indiquée, produit de formule (V) que l'on traite par l'anhydride trifluoroacétique pour obtenir un produit de formule (VI) :

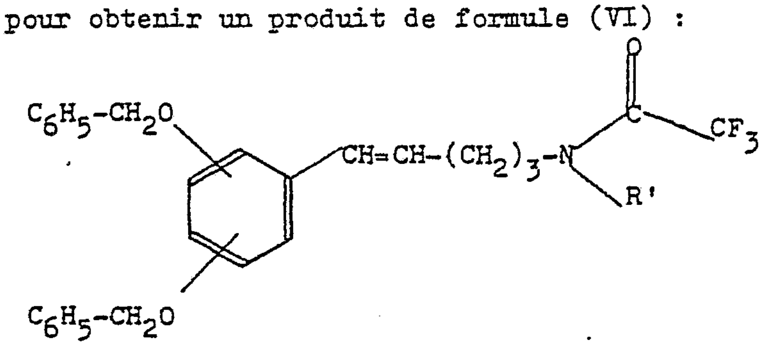

(VI)

dans laquelle R' a la signification déjà indiquée, produit de formule (VI) que l'on traite par la N-bromo succinimide pour obtenir un produit de formule (VII) :

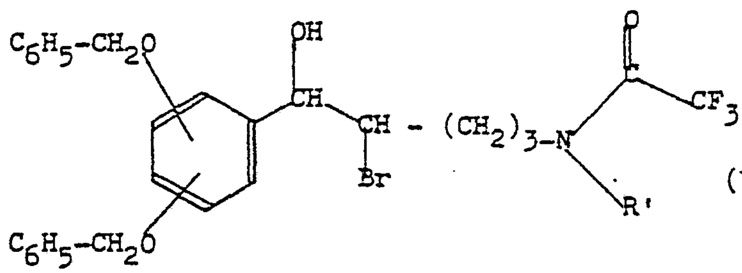

(VII)

dans laquelle R' a la signification déjà indiquée, produit de formule (VII) que l'on traite par un agent alcalin, pour obtenir un produit de formule (VIII) :

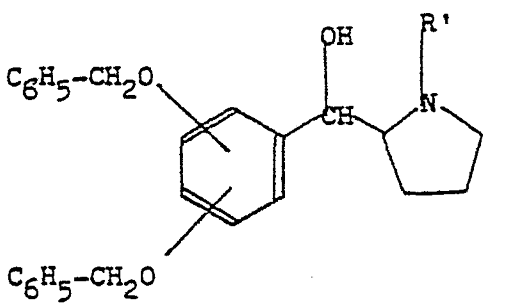

(VIII)

dans laquelle R' a la signification déjà indiquée, sous forme d'un mélange d'isomères A ($VIII_A$) et B ($VIII_B$) que l'on sépare si désiré, produit de formule (VIII), ($VIII_A$) ou ($VIII_B$) que l'on soumet à une hydrogénolyse pour obtenir le produit de formule (I) recherché, sous forme d'un mélange d'isomères A et B que l'on sépare, si désiré, ou sous forme d'isomère A ou B, produit

de formule (I) que, si désiré,

- soit l'on salifie,
- soit, lorsque R représente un atome d'hydrogène, l'on fait réagir avec un halogénure de formule (IX) :

Hal-R" (IX)

dans laquelle Hal représente un atome de chlore, de brome ou d'iode et R" représente un radical alcoyle renfermant de 1 à 5 atomes de carbone, pour obtenir un produit de formule (I') :

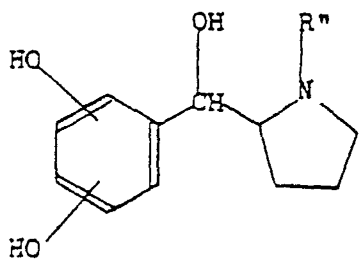

(I')

dans laquelle R" a la signification déjà indiquée, correspondant à un produit de formule (I) dans laquelle R a la définition de R", sous forme d'un mélange d'isomères A et B, que l'on sépare, si désiré, ou sous forme d'isomère A ou B, produit de formule I'que, si désiré, l'on salifie.

Dans des conditions préférentielles de mise en oeuvre de l'invention, le procédé de préparation ci-dessus décrit est caractérisé en ce que :

- l'on fait réagir le bromure de 4-bromobutyl triphénylphosphonium avec l'aldéhyde de formule (II) en les ajoutant, à l'état solide, à l'hydrure de sodium dans un mélange de tétrahydrofurane et de diméthyl sulfoxyde;
- l'on fait réagir le produit de formule (III) avec l'amine de formule (IV) à la température de 120°C pendant 8 à 12 heures;
- l'on traite le produit de formule (V) à l'aide d'anhydride trifluoroacétique à température ambiante pendant 12 à 16 heures;
- l'on traite le produit de formule (VI) par le N-bromo succinimide en présence d'une solution aqueuse d'acide sulfurique, d'abord à froid aux environs de 12°C, puis à température ambiante;
- l'agent alcalin utilisé pour la cyclisation du produit de formule (VII) est une solution aqueuse de soude, mais il est

aussi possible d'utiliser une solution aqueuse de potasse;

- la cyclisation du produit de formule (VII) est effectuée sous atmosphère inerte dans le dioxane, au reflux du mélange réactionnel;
- l'hydrogénolyse catalytique du produit de formule (VIII) est effectuée dans le méthanol à 50°C en présence de carbone palladié;
- la séparation optionnelle des mélanges d'isomères A et B de produits de formule VIII et I est effectuée par chromatographie.

L'invention a aussi pour objet un procédé de préparation des dérivés, tels que définis par la formule (I) ci-dessus, ainsi que de leurs sels, dans laquelle les hydroxyles sont en position 2,4 ou 2,5 ou 3,4, caractérisé en ce que l'on traite à l'aide de brome un produit de formule (X) :

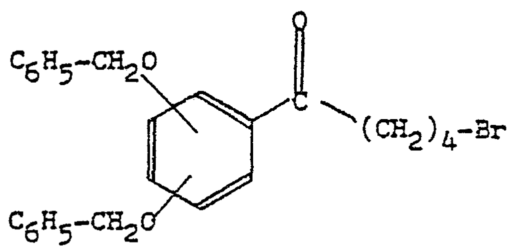

(X)

dans laquelle les radicaux benzyloxy sont en position 2,4 ou 2,5 ou 3,4, pour obtenir un produit de formule (XI):

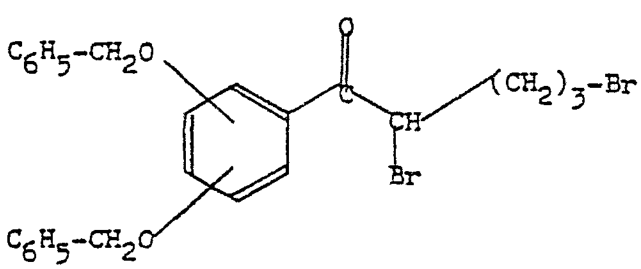

(XI)

que l'on cyclise à l'aide d'une amine de formule (IV)

$R'-NH_2$ (IV)

dans laquelle R' représente un radical benzyle ou un radical alcoyle renfermant de 1 à 5 atomes de carbone, pour obtenir un produit de formule (XII):

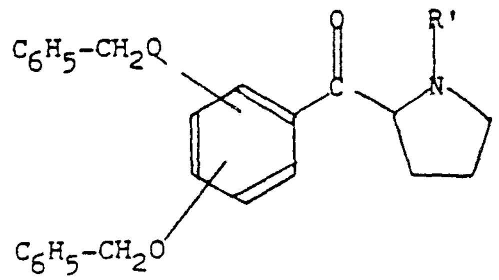

(XII)

dans laquelle R' a la signification déjà indiquée, produit de formule (XII) que l'on réduit pour obtenir un produit de formule (XIII) :

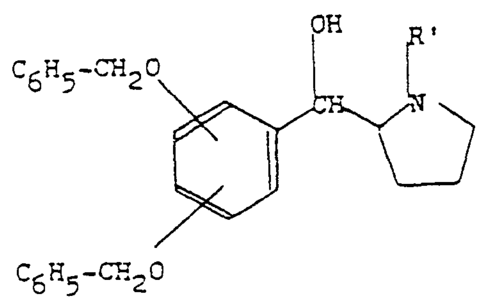

(XIII)

dans laquelle R' a la signification déjà indiquée, sous forme d'un mélange d'isomères A ($XIII_A$) et B ($XIII_B$) que l'on sépare si désiré, produit de la formule (XIII), ($XIII_A$) ou ($XIII_B$) que l'on soumet à une hydrogénolyse, pour obtenir le produit de formule (I) recherché, sous forme d'un mélange d'isomères A et B, que l'on sépare, si désiré, ou sous forme d'isomère A ou B, produit de formule (I) que, si désiré,

- soit l'on salifie,
- soit, lorsque R représente un atome d'hydrogène, l'on fait réagir avec un halogénure de formule (IX) :

Hal-R" (IX)

dans laquelle Hal et R" ont la signification déjà indiquée, pour obtenir un produit de formule (I") :

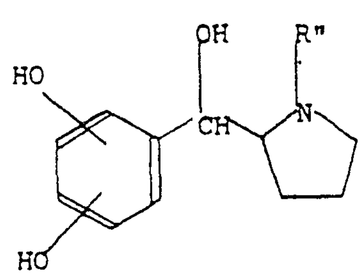

(I")

dans laquelle R" a la signification déjà indiquée, correspondant à un produit de formule (I) dans laquelle R a la définition de R" et les substituants OH sont en position 2,4 ou 2,5 ou 3,4 sous forme d'un mélange d'isomères A et B, que l'on sépare, si désiré, ou sous forme d'isomère A ou B, produit de formule (I") que, si désiré, l'on salifie.

Dans des conditions préférentielles de mise en oeuvre de l'invention, le procédé de préparation des dérivés de formule (I) ci-dessus décrit est caractérisé en ce que :

- le produit de formule (X) est traité par une solution de brome dans l'acide acétique et le mélange réactionnel est rapidement traité par de l'eau glacée après l'addition du brome,
- la cyclisation du produit de formule (XI) est effectuée dans le toluène, mais il est possible d'utiliser d'autres solvants comme le benzène ou le xylène;
- le produit de formule (XII) est réduit à l'aide de borohydrure de sodium dans le méthanol, selon que l'on veut de préférence obtenir l'isomère A du produit de formule (I) cherché, ou à l'aide de dihydrure de diéthylsodiumaluminium dans le toluène, selon que l'on veut de préférence obtenir l'isomère B du produit de formule (I) cherché;
- l'hydrogénolyse catalytique du produit de formule (XIII) est effectuée à 50°C, en présence de carbone palladié;
- la séparation optionnelle des isomères A et B des produits de formules (XIII) et (I) est obtenue par chromatographie

L'invention a aussi pour objet un procédé de préparation des dérivés, tels que définis par la formule (I) ci-dessus, sous la forme de l'isomère A, ainsi que de leurs sels, procédé qui consiste à traiter un produit de formule (X) :

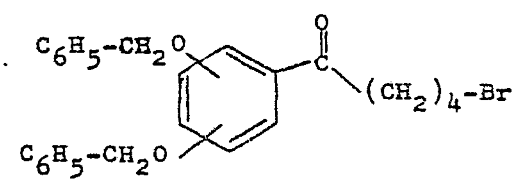

(X)

par le brome, pour obtenir un produit de formule (XI) :

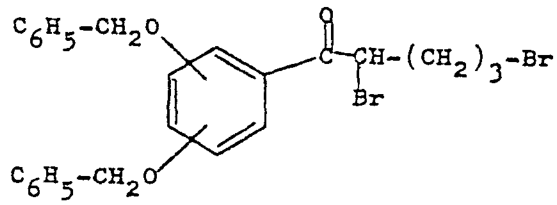

(XI)

et qui est caractérisé en ce que l'on traite ledit produit de formule (XI) par un agent réducteur, pour obtenir un produit de formule (XIV) :

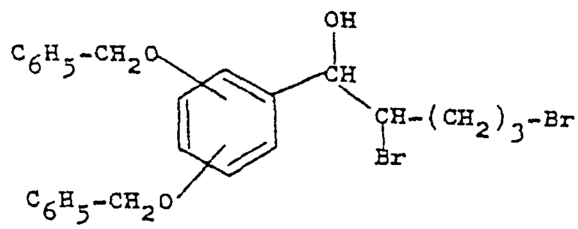

(XIV)

que l'on traite par un alcoolate alcalin, pour obtenir un produit de formule (XV) :

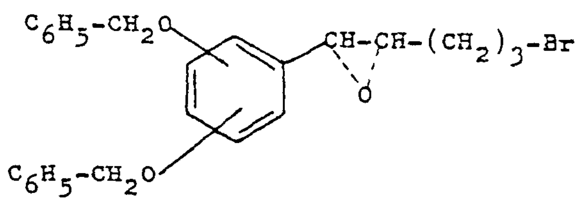

(XV)

que l'on traite par une amine de formule (IV) :

$R'-NH_2$ (IV)

dans laquelle R' représente un radical benzyle ou un radical alcoyle renfermant de 1 à 5 atomes de carbone, pour obtenir un produit de formule (XIII) telle que définie précédemment, sous forme d'isomère A, produit que l'on soumet à une hydrogénolyse, pour obtenir le produit de formule (I) correspondant, sous forme d'isomère A, produit que, si désiré,

- soit l'on salifie,
- soit, lorsque R représente un atome d'hydrogène, l'on fait réagir avec un halogénure de formule (IX) :

Hal-R" (IX)

dans laquelle Hal et R" ont la signification déjà indiquée, pour obtenir un produit de formule (I"') :

BAD ORIGINAL

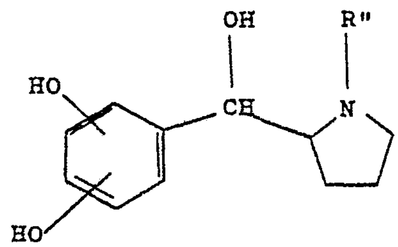

(I''')

dans laquelle R" a la signification déjà indiquée, correspondant à un produit de formule I dans laquelle R a la définition de R" sous forme d'isomère A, produit de formule (I") que, si désiré, l'on salifie.

Dans des conditions préférentielles de mise en oeuvre de l'invention, le procédé de préparation des dérivés de formule (I) ci-dessus décrit est caractérisé en ce que :

- l'agent réducteur est le borohydrure de sodium et l'on opère dans le méthanol ;
- l'époxydation par l'alcoolate alcalin est effectuée par le teramylate de sodium au sein du tétrahydrofuranne ;
- le traitement par l'amine de formule (IV) est effectué en l'absence de solvant ;
- l'hydrogénolyse du produit de formule XIII est effectuée à 50°C, en présence du carbone palladié.

L'invention a aussi pour objet un procédé de préparation des dérivés, tels que définis par la formule (I) ci-dessus, sous la forme de l'isomère A, ainsi que de leurs sels, procédé qui consiste à faire réagir le bromure de 4-bromobutyl triphényl phosphonium avec un aldéhyde de formule (II) :

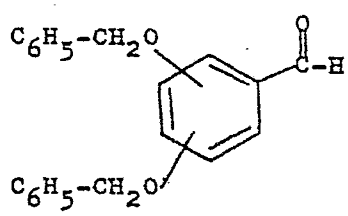

(II)

pour obtenir un produit de formule (III) :

BAD ORIGINAL

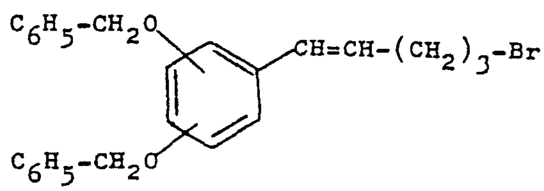

(III)

sous forme de l'isomère cis, et qui est caractérisé en ce que l'on traite ledit produit de formule III par un agent d'époxydation, pour obtenir un produit de formule (XV), telle que définie précédemment, et poursuit la synthèse comme décrit précédemment, à partir de ce produit de formule XV.

Dans des conditions préférentielles de mise en oeuvre de l'invention, le procédé de préparation des dérivés de formule I ci-dessus décrit est caractérisé en ce que l'agent d'époxydation utilisé est l'eau oxygénée.

Les dérivés de formule (I) présentent un caractère basique. On peut avantageusement préparer les sels d'addition des dérivés de formule (I) en faisant réagir, en proportions sensiblement stoechiométriques, un acide minéral ou organique avec ledit dérivé de formule (I). Les sels peuvent être préparés sans isoler les bases correspondantes.

Les dérivés objet de la présente invention, possèdent de très intéressantes propriétés pharmacologiques; ils sont doués notamment de remarquables propriétés bronchodilatatrices et hypotensives ainsi que des propriétés antidépressives.

Ces propriétés sont illustrées plus loin dans la partie expérimentale.

Ces propriétés justifient l'utilisation des dérivés de l'α-phényl 2-pyrrolidineméthanol, ainsi que de leurs sels, à titre de médicaments.

La présente demande a ainsi également pour objet l'application, à titre de médicaments, des dérivés de l'α-phényl 2-pyrrolidineméthanol, tels que définis par la formule (I), ainsi que de leurs sels d'addition avec les acides pharmaceutiquement acceptables.

BAD ORIGINAL

Parmi les médicaments, objet de l'invention, on retient, de préférence, les médicaments caractérisés en ce qu'ils sont constitués par les nouveaux dérivés de l'α-phényl 2-pyrrolidinéméthanol répondant à la formule (I), dans laquelle les groupements hydroxyles sont en positions 3 et 4 ou 3 et 5, et R représente un atome d'hydrogène, ainsi que par leurs sels d'addition avec les acides pharmaceutiquement acceptables.

Parmi ceux-ci on retient notamment le 4-/α-hydroxy (pyrrolidin-2-yl)méthyl/1,2-benzène diol sous ses formes optiquement actives ou racémiques, ainsi que ses sels d'addition avec les acides pharmaceutiquement acceptables.

Parmi ces derniers, on retient tout particulièrement, l'oxalate acide de l'isomère A du 4-/α-hydroxy(pyrrolidin-2-yl)méthyl/1,2-benzène diol sous ses formes optiquement actives ou racémiques.

Les médicaments selon l'invention trouvent leur emploi, par exemple, dans le traitement de l'asthme, des bronchites asthmatiformes, des bronchites chroniques, de l'hypertension artérielle sous toutes ses formes : permanente légère, modérée ou sévère ou encore dans le traitement des états dépressifs.

La dose usuelle, variable selon le dérivé utilisé, le sujet traité et l'affection en cause peut être, par exemple, de 1 mg à 50 mg par jour, par voie orale chez l'homme.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive, parentérale ou locale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations

BAD ORIGINAL

injectables, les aérosols ; elles sont préparées selon les méthodes usuelles. Le ou les principes actifs peuvent y être incorporés à des excipients habituellement employés, dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale ; les dérivés paraffiniques, les glycols, les divers agents mouillants, dispersants ou émulsifiants, les conservateurs.

Les dérivés de formule (II) lorsqu'ils ne sont pas connus peuvent être préparés par benzylation d'un acide de formule (XIV) :

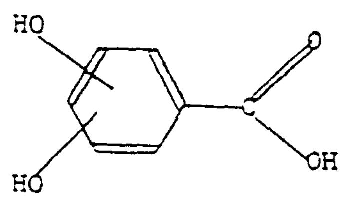

(XIV)

puis transformation, selon les procédés classiques, de l'acide dibenzylé ainsi obtenu, en chlorure d'acide correspondant, et enfin selon la méthode de Rosenmund, réduction catalytique du chlorure d'acide, pour obtenir l'aldéhyde désiré.

Les dérivés de formule (X) dans laquelle les radicaux benzyloxy sont en position 2,4 ou 2,5 ou 3,4, lorsqu'ils ne sont pas connus, peuvent être préparés par réaction de Friedel et Crafts entre un dihydroxybenzène dont les radicaux hydroxy se trouvent en position 1,3 ou 1,4 ou 1,2 et l'acide 5-bromovalérique, puis benzylation à l'aide de bromure de benzyle, en présence d'un agent alcalin. Un exemple d'une telle préparation est donné ci-après dans la partie expérimentale.

Le procédé de l'invention permet de préparer des produits industriels nouveaux, à savoir, notamment, les dérivés de formules (VI),(VII),(VIII),(XI),(XII),(XIV) et (XV), telles que définies précédemment.

BAD ORIGINAL

Il va être donné maintenant, à titre non limitatif, des exemples de mise en oeuvre de l'invention.

Exemple 1 : Oxalate acide de 4-[α-hydroxy(pyrrolidin-2-yl) méthyl]1,2-benzène diol (isomère A).

Stade A : 1,2-bis[(phénylméthyl)oxy]4-(5-bromo pent-1-èn-1-yl) benzène (isomère cis)

On ajoute progressivement en une heure trente à une suspension de 5,28 g d'hydrure de sodium dans 350 cm3 de tétrahydrofuranne et 350 cm3 de diméthylsulfoxyde, à la température de 5°C, un mélange de 47,8 g de bromure de 4-bromobutyl triphényl phosphonium (Mondon : Annalen der Chemie 603 - 115 (1957) et 28,6 g de 3,4-dibenzyloxybenzaldéhyde, agite pendant 27 heures à température ambiante, amène à sec par chauffage à 70-80°C sous pression réduite, reprend la masse gélatineuse obtenue par 700 cm3 d'éther, agite une heure, laisse reposer une nuit, filtre et amène à sec. On purifie par redissolution dans le benzène, puis passage rapide sur 700 g de silice et obtient après évaporation du solvant 35,60 g du produit cherché.

Stade B : Chlorhydrate de N-[5-[3,4-bis[(phénylméthyl)oxy] phényl[pent-5-èn-1-yl]benzène éthanamine (isomère cis)

On fait agir pendant 10 heures à 120°C, 29,25 g du produit obtenu au stade précédent, avec 72 cm3 de benzylamine, puis chasse l'excès d'amine à 100°C sous pression réduite, reprend le résidu par 500 cm3 d'éther, lave à l'eau, sèche, évapore et obtient 36,85 g d'une huile épaisse que l'on met en solution dans 300 cm3 d'éther. On ajoute 22 cm3 d'acide chlorhydrique 5N en solution alcoolique, essore le chlorhydrate, lave à l'éther, sèche sous pression réduite et purifie dans le chlorure de méthylène. On obtient 30 g de produit attendu fondant à 150°C.

Stade C : N-[5-[3,4-bis[(phénylméthyl)oxy]phényl[pent-5-èn-1-yl]N-phénylméthyl 2,2,2-trifluoroacétamide (isomère cis)

On fait agir 30 g du chlorhydrate obtenu au stade précédent dans 100 cm3 de chlorure de méthylène, avec 50 g d'anhydride trifluoroacétique en présence de 31,8 g de carbonate de sodium anhydre. On agite une nuit à température ambiante, lave par une solution aqueuse à 10 % de bicarbonate de potassium, sèche et évapore sous pression réduite la phase organique et obtient 28,5 g du produit cherché.

chromatographie en couche mince sur silice : éluant : benzène-acide acétique (9/1) Rf. = 0,45.

Stade D : N-[5-[3,4-bis[(phénylméthyl)oxy]phényl[4-bromo 5-hydroxypentyl]N-(phénylméthyl)2,2,2-trifluoroacétamide

On ajoute sous atmosphère inerte, à une solution de 5,6 g du produit obtenu au stade précédent dans 100 cm3 de dioxane à 10 - 15°C, 7,2 cm3 d'une solution aqueuse d'acide sulfurique de densité 1,83 puis, en 15 minutes, une solution de 1,9 g de N-bromo succinimide dans 25 cm3 de dioxane. Après 15 minutes, on laisse revenir à température ambiante, maintient sous agitation pendant une nuit, dilue par 400 cm3 d'éther, lave à l'eau, sèche, traite au charbon actif, amène à sec la phase organique et obtient 7,8 g de produit cherché.

Stade E : α-[3,4-bis[(phénylméthyl)oxy]phényl]1-(phénylméthyl) 2-pyrrolidine méthanol (isomère A).

On porte au reflux dans 200 cm3 de dioxane, 18,5 g de produit obtenu au stade précédent avec 87 cm3 de soude N pendant une heure 30 sous atmosphère inerte, laisse reposer une nuit, reprend à l'éther, lave à l'eau, évapore, recueille 10,45 g d'une huile que l'on chromatographie sur colonne de silice en éluant à l'aide d'un mélange chlorure de méthylène-méthanol (95-5). Ainsi on isole trois fractions, recueille la fraction la moins mobile consistant en 2,4 g d'une huile épaisse correspondant à l'isomère A du produit recherché que l'on cristallise dans l'éther isopropylique. On obtient des cristaux blancs fondant à 72°C. La fraction de mobilité moyenne correspond à l'isomère A contenant des traces de l'isomère B.

| Analyse : | | Calculé | | Trouvé |
|---|---|---|---|---|
| $C_{32}H_{33}NO_3$ = 479,6 | C% | 80,13 | C% | 80,2 |
| | H% | 6,93 | H% | 7,1 |
| | N% | 2,92 | N% | 2,8 |

Stade F : Oxalate acide du 4-[α-hydroxy(pyrrolidin-2-yl) méthyl 1,2-benzène diol (isomère A).

On agite sous pression de 0,5 bar d'hydrogène à 50°C pendant 40 minutes, 4,095 g de produit obtenu au stade précédent dans 55 cm3 de méthanol avec 0,82 g de carbone palladié.

Préparation de l'oxalate acide

On met ensuite le mélange ci-dessus sous atmosphère d'azote, ajoute 25 cm3 d'une solution méthanolique d'acide oxalique à 4 g pour 100 cm3, agite pendant quelques minutes, filtre, évapore le solvant sous pression réduite, sèche à 70°C et recueille 2,7 g de produit blanc que l'on lave à l'éthanol puis à l'éther. On sèche et obtient 2,35 g de produit correspondant au produit cherché fondant avec décomposition à 220-225°C.

Analyse

$C_{13}H_{17}NO_7$ = 299,28

| | Calculé | | Trouvé |
|---|---|---|---|
| C% | 52,17 | C% | 52,0 |
| H% | 5,73 | H% | 5,8 |
| N% | 4,68 | N% | 4,4 |

Exemple 2 : Oxalate acide du 4-/α-hydroxy(pyrrolidin-2-yl) méthyl/1,2-benzène diol (isomère A).

Stade A : 2,5-dibromo 1-/3,4bis (phénylméthyloxy)phényl/1-pentanone

On dissout dans 350 cm3 d'acide acétique, 38,47 g de 5-bromo 1-/3,4-bis (phénylméthyloxy)phényl/1-pentanone, par chauffage à 60°C, refroidit, introduit, goutte à goutte, en refroidissant, une solution de 14 g de brome dans 40 cm3 d'acide acétique, agite encore pendant 5 minutes après la cristallisation obtenue en fin d'addition, ajoute alors un litre d'eau glacée, agite 5 minutes, essore, lave à l'eau les cristaux, sèche sous pression réduite à 60°C, recristallise dans le cyclohexane les 43,2 g de cristaux obtenus et recueille 38 g du produit attendu fondant à 100°C.

Analyse

$C_{25}H_{24}Br_2O_3$ = 532,28

| | Calculé | | Trouvé |
|---|---|---|---|
| C% | 56,41 | C% | 56,3 |
| H% | 4,54 | H% | 4,6 |
| N% | 30,03 | N% | 30,2 |

Stade B : /3,4-bis/(phénylméthyl)oxy/phényl/ /1-(phénylméthyl) 2-pyrrolidinyl/méthanone

On porte au reflux dans 100 cm3 de toluène anhydre pendant 4 heures, 21,28 g de produit obtenu au stade précédent avec 14,12g de benzylamine, refroidit, élimine par filtration le précipité formé et récupère le filtrat utilisé tel quel

au stade suivant.

Stade C : α/3,4-bis/(phénylméthyl)oxy/phényl/1-(phénylméthyl) 2-pyrrolidineméthanol (isomère A).

On agite pendant 17 heures à 65°C, le filtrat obtenu au stade précédent, sous atmosphère inerte avec 5,12 g de borohydrure de sodium à 95 %, 40 cm3 de soude 2N, 200 cm3 d'eau et 3 g de bromure de cétyltriméthylammonium. On ajoute après refroidissement 500 cm3 d'éther, décante, extrait la phase aqueuse à l'éther, lave la phase organique à l'eau salée, sèche, chasse sous pression réduite les solvants. On recueille 21,35 g d'huile jaune que l'on chromatographie sur silice en éluant avec un mélange chlorure de méthylène-méthanol (97-3). On obtient trois fractions et isole la fraction la moins mobile correspondant à l'isomère A.

L'isomère A récupéré, recristallisé dans 2,5 volumes d'éther isopropylique, conduit à 5,515 g de produit pur fondant à 73-74°C.

| Analyse : | | Calculé | | Trouvé |
|---|---|---|---|---|
| $C_{32}H_{33}NO_3$ = 479,6 | C% | 80,13 | C% | 80,2 |
| | H% | 6,93 | H% | 7,1 |
| | N% | 2,92 | N% | 2,8 |

RMN ($CDCl_3$)

constante de couplage φ - C (H) - C (H) - N< J ≃ 5 Hz

254 - 259 Hz φ - C (H) - O-

192 - 205 Hz }
215 - 223 Hz } N - $CH_2$ - φ

240 HZ OH mobile

105 Hz (massif) les $CH_2$ en β de l'azote

130 à 190 Hz $CH_2$-N et C (H)- N

304 Hz O - $CH_2$ - φ

410 à 440 Hz aromatiques

Stade D : Oxalate acide du 4-/α-hydroxy(pyrrolidin-2-yl) méthyl/1,2-benzène diol (isomère A)

On traite selon le procédé indiqué au stade F de l'exemple 1, le produit obtenu au stade précédent.

La 5-bromo 1-/3,4-bis(phénylméthyloxy)phényl/1-pentanone de départ du stade A peut être préparé de la façon suivante :

a) Préparation de la 5-bromo 1-(3,4-dihydroxyphényl)1-pentanone.

On fait barboter du trifluorure de bore dans une suspension

de 91,2 g de pyrocatéchol dans 695 cm3 de trichloréthylène avec 150 g d'acide 5-bromo valérique (Isenberg et Coll. Can. J. 40 831 (1962), puis chauffe pendant 2 heures 40 à 67°C, après barbotage de 154 g de trifluorure de bore, on laisse sous agitation, revenir lentement à température ambiante pendant une nuit. On élimine la phase supérieure des 2 phases ainsi obtenues, lave par 2 fois 100 ml de trichloréthylène, verse dans 650 cm3 d'eau glacée, agite pendant une heure, essore les cristaux, lave à l'eau, sèche et récupère 186 g de cristaux que l'on recristallise dans le toluène. On obtient 163 g du produit cherché fondant à 110-115°C.

b) 5-bromo 1-/3,4-bis(phénylméthyloxy) phényl/1-pentanone

On agite sous atmosphère inerte une solution de 30,6 g du produit obtenu au stade précédent dans 390 cm3 d'acétone avec 155 g de carbonate de potassium. On introduit goutte à goutte en 2 heures à 23°-25°C, 39,1 g de bromure de benzyle en solution dans 300 cm3 d'acétone, agite pendant 21 heures 30, filtre, évapore le filtrat sous pression réduite et recueille 51 g de produit brut. On purifie par chromatographie sur silice par le chlorure de méthylène, et obtient après séchage sous pression réduite, 43 g du produit désiré qui, recristallisé dans le cyclohexane fond à 71°C.

Exemple 3 : Chlorhydrate du 4-/α-hydroxy(pyrrolidin-2-yl) méthyl/1,2-benzène diol (isomère B)

Stade A : α/3,4-bis/(phénylméthyl)oxy/phényl/ /1-(phénylméthyl)2-pyrrolidinyl/méthanone

On porte au reflux dans 150 cm3 de toluène anhydre 22,08 g de produit tel qu'obtenu au stade A de l'exemple 2, laisse descendre à la température à 100°C, ajoute en 4 minutes une solution de 15 cm3 de benzylamine dans 25 cm3 de toluène, agite encore 2 heures 30 au reflux, refroidit, élimine par filtration le précipité formé et utilise tel quel le filtrat pour le stade suivant.

Stade B : α/3,4-bis/(phénylméthyl)oxy/phényl/ 1-(phénylméthyl) 2-pyrrolidineméthanol (isomère B).

On agite à 20°C sous atmosphère inerte le filtrat obtenu au stade précédent avec 61,5 cm3 de solution toluénique à 1,35 mole de dihydrure de diéthyl sodium aluminium par litre que l'on a ajouté en 5 minutes. On agite alors pendant

18 heures 30 à 20-23°C puis refroidit à 8°C, ajoute en 30 minutes 15 g de glace en petits morceaux, puis introduit à 10°C, 250 cm3 de lessive de soude. On agite 30 minutes, décante, extrait la phase aqueuse au toluène, lave à l'eau la phase organique, sèche, chasse sous pression réduite les solvants, recueille 20,8 g d'huile jaune que l'on chromatographie sur silice (éluant : chlorure de méthylène-méthanol (97-3). On obtient 4 fractions, isole la fraction la plus mobile correspondant à l'isomère B et l'amène à sec. On recristallise le produit dans 20 cm3 d'éther isopropylique et obtient 7,475 g de produit recherché, pur, fondant à 82°C.

<u>Analyse</u>

$C_{32}H_{33}NC_3$ = 479,6

| | Calculé | | Trouvé | |
|---|---|---|---|---|
| | C% | 80,13 | C% | 80,0 |
| | H% | 6,93 | H% | 7,0 |
| | N% | 2,92 | N% | 2,8 |

<u>RMN ($CDCl_3$)</u>

<u>Constante</u> de couplage (J ≃ 3 Hz) φ - C(O) (H) - C (H) - N<

| | |
|---|---|
| 284 - 287 Hz | φ - C (H) - O |
| 241 - 254 Hz<br>197 - 210 Hz | N-$CH_2$ - φ |

environ de 95 Hz (massif) les $CH_2$ en β de l'azote

| | |
|---|---|
| 125 à 200 Hz | $CH_2$ N et C (H) -N |
| 307 à 309 Hz | O - $CH_2$ - φ |
| 410 à 450 Hz | aromatique |

<u>Stade C : Chlorhydrate du 4-/α-hydroxy(pyrrolidin-2-yl)méthyl/ 1,2-benzènediol (isomère B).</u>

On agite pendant 45 mn, sous atmosphère d'hydrogène, à la température de 54°C, 4 g du produit obtenu au stade précédent, dans 52 cm3 de diméthylformamide avec 0,650 g de carbone palladié à 9,8%.

On refroidit le mélange à 25°C, ajoute 2,5 cm3 de solution éthanolique d'acide chlorhydrique 6,6 N, filtre, évapore les solvants, sèche à 54°C sous pression réduite et recueille 3,5 g d'huile jaune brun que l'on dissout dans 30 cm3 d'eau à 60°C sous atmosphère inerte. On filtre la solution obtenue, sous atmosphère inerte, ajoute 2 cm3 d'ammoniaque 20°Bé, glace, laisse cristalliser pendant une heure 30, lave les cristaux à l'eau, sèche sous pression réduite et recueille

1,47 g du produit cherché sous forme non salifiée fondant à 250°C.

Analyse : $C_{11}H_{15}NO_3$ = 209,2

| | | | | |
|---|---|---|---|---|
| Calculé : | C % | 63,14 | H % 7,23 | N % 6,69 |
| Trouvé : | | 63,0. | 7,4 | 6,4. |

Préparation du chlorhydrate

On agite 3,285 g de produit tel qu'obtenu ci-dessus en solution dans 13 cm3 d'alcool absolu avec 2,6 cm3 de solution éthanolique d'acide chlorhydrique 6,6 N pendant une nuit sous atmosphère inerte. On glace pendant une heure, essore, lave les cristaux avec un mélange glacé d'éthanol et d'éther, sèche sous vide et obtient 3,41 g du chlorhydrate cherché fondant à 250°C.

Analyse : $C_{11}H_{16}ClNO_3$ = 245,7

| | | | | |
|---|---|---|---|---|
| Calculé : | C % 53,77 | H % 6,56 | N % 5,70 | Cl % 14,43 |
| Trouvé : | 53,6 | 6,7 | 5,5 | 14,2. |

Exemple 4 : Oxalate acide du 4-/α-hydroxy(pyrrolidin-2-yl) méthyl /1,2-benzène diol (isomère A).

Stade A : 3,4-bis(phénylméthoxy)α-(1,4-dibromobutyl)benzène méthanol.

On met en suspension 0,532 g de 2,5-dibromo 1-/3,4-bis (phénylméthoxy)phényl/1-pentanone (obtenu au stade A de l'exemple 2) dans 10,6 cm3 de méthanol. On ajoute à 0,+5°C 0,08 g de borohydrure de sodium. On agite pendant une heure 15 minutes à 0,+5°C, verse le mélange sur une solution aqueuse 1N d'acide chlorhydrique à 0°C, extrait à l'éther, sèche et évapore le solvant. On obtient 0,472 g de produit attendu que l'on utilise tel quel pour le stade suivant.

Stade B : 2-/3,4-bis(phénylméthoxy)phényl/3-(1-bromopropyl) oxiranne.

On met en suspension 0,05 g d'hydrure de sodium à 56 % dans l'huile minérale dans 1 cm3 de tétrahydrofuranne puis ajoute 0,19cm3 d'alcool teramylique dans 1cm3 de tétrahydrofurane. On laisse monter la température jusqu'au reflux puis refroidit à 20°C. On ajoute 0,534g du produit obtenu comme décrit au stade A, maintient sous agitation pendant 45 minutes, verse sur une solution aqueuse d'acide chlorhydrique, extrait au chlorure de méthylène, sèche la phase organique et évapore le solvant. On obtient 0,393 g de produit attendu, utilisé tel quel pour le stade suivant.

Stade C : α-/3,4-bis(phénylméthoxy)phényl/1-(phénylméthyl)

2-pyrrolidin méthanol (isomère A)

On agite pendant 4 heures un mélange de 0,361 g du produit obtenu au stade B et 0,343 g de benzylamine, verse sur une solution aqueuse de soude, extrait au chlorure de méthylène, sèche et évapore le solvant. On obtient 0,3 g de produit attendu identique au produit obtenu à l'exemple 1 (stade E) et à l'exemple 2 (stade C).

Stade D : Oxalate acide du 4-/α-hydroxy(pyrrolidin-2-yl) méthyl/1,2-benzène diol (isomère A)

On opère au départ du produit obtenu au stade C ci-dessus, comme décrit à l'exemple 1 ou 2 et obtient le produit attendu.

Exemple 5 : Oxalate acide du 4-/α-hydroxy (pyrrolidin-2-yl) méthyl/1,2-benzène diol (isomère A)

Stade A : 2-/3,4-bis(phénylméthoxy)phényl/3-(1-bromopropyl) oxiranne

On introduit à 0°C, 1,55 g d'acide métachloroperbenzoïque en solution dans 20 cm3 de chlorure de méthylène, dans une solution de 1 g de 1,2-bis(phénylméthoxy)4-(5-bromopent-1-èn 1-yl)benzène obtenu comme décrit au stade A de l'exemple 1, dans 10 cm3 de chlorure de méthylène. On maintient pendant 5 heures à 20°C, filtre, lave le filtrat avec une solution aqueuse de bicarbonate de sodium, sèche et évapore le solvant. On obtient le produit attendu, utilisé tel que pour le stade suivant.

Stade B : Oxalate acide du 4-/α-hydroxy (pyrrolidin-2-yl) méthyl/1,2-benzène diol

On opère au départ du produit obtenu au stade A ci-dessus comme décrit au stade C de l'exemple 4 puis comme décrit à l'exemple 1 ou 2 et obtient le produit attendu.

Exemple 6 :

On a préparé des comprimés répondant à la formule :

- Oxalate acide du 4-/α-hydroxy(pyrrolidin-2-yl)méthyl/ 1,2-benzène diol (isomère A) ...................... 2 mg
- Excipient q.s. pour un comprimé terminé à ....... 100 mg

(Détail de l'excipient : lactose, amidon, talc, stéarate de magnésium).

Exemple 7 :

On a préparé des comprimés répondant à la formule :

- Chlorhydrate du 4-/α-hydroxy (pyrrolidin-2-yl) méthyl/

1,2-benzène diol (isomère B).................... 5 mg
(Détail de l'excipient : lactose, amidon, talc, stéarate de magnésium).

Exemple 8 :

On a préparé des aérosols délivrant des doses contenant chacune :

- Oxalate acide du 4-/α-hydroxy (pyrrolidin-2-yl)méthyl/ 1,2-benzène diol (isomère A)...................... 0,1 mg
- Emulsifiant.................................... 0,15 mg
- Propulseur..................................... 50 mg.

Etude pharmacologique

1) Action sur la trachée isolée de cobaye

On prélève les trachées de cobaye que l'on vient de sacrifier par un coup sur la nuque, les trachées sont découpées en spirale et suspendues dans une solution de Krebs Henseleit, à la température de 37°C dans laquelle barbote un mélange de 95 % d'oxygène et 5 % de gaz carbonique.

On ajoute dans la solution du carbachol comme agoniste pour provoquer des contractions.

Le produit de l'exemple 1 à la concentration de $3.10^{-6}$M, antagonise de 100 % les contractions provoquées par le carbachol à la concentration de 0,4γ/ml.

2) Action sur le bronchospasme provoqué par l'histamine chez le cobaye

Suivant la méthode de Konzett et Rossler Arch. exp. Path. Pharmakol. 195 71 (1940), de l'histamine est administrée à une dose variant de 2 à 10µg/kg i.v. selon la sensibilité de l'animal.

L'enregistrement de la pression produite par l'air expulsé est effectué à l'aide d'un microdynamomètre Apelab.

Le produit à tester est injecté en solution dans le sérum physiologique par voie i.v. une minute avant l'histamine.

On enregistre un éventuel effet du produit seul et son action antagoniste vis-à-vis de l'agent constricteur.

L'activité antagoniste est exprimée en pourcentage de protection par rapport au bronchospasme initial dû à l'histamine.

Injecté à la dose de 1µg/kg, le produit de l'exemple 1 a inhibé de 52 % la broncho constriction à l'histamine et le produit de l'exemple 3 à cette même dose, l'a inhibée de 17%.

3) Déterminiation de l'activité hypotensive

L'activité hypotensive a été étudiée sur des rats mâles de souche Sprague Dawley S.P.F. pesant 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle, après administration du produit testé par rapport à la pression artérielle initiale ainsi que le temps pendant lequel se manifestent ces variations.

| Produit de l'exemple | Doses (mg/kg) | Variations % de la pression artérielle | Durée d'action (minutes) |
|---|---|---|---|
| 1 | 1 | -35 | >65 |

4) Action antihypertensive chez le chien éveillé

L'étude de l'action antihypertensive a été effectuée sur le chien Beagle pesant de 12 kg à 14 kg rendu hypertendu par enveloppement des 2 reins par de la cellophane. Selon la technique décrite par Irvine H.Page dans Science (1939) 89 p 273 - 274.

Le produit a été administré per os à la dose de 10 mg/kg.

La pression artérielle a été mesurée à la queue du chien au moyen d'un manchon pneumatique relié à un transducteur électronique de pression. La pression a été mesurée avant et après administration du produit.

Le tableau ci-après indique les variations exprimées en pourcentages de la pression artérielle, après administration du produit par rapport à la pression témoin initiale.

BAD ORIGINAL

| Produit de l'exemple | Dose administrée mg/kg | Variation % de la pression artérielle | | | |
|---|---|---|---|---|---|
| | | 1 er jour | | | |
| | | 1 heure après l 1 ère adminis-tration | 3 heures après la 1 ère adminis-tration | 6 heures après la 1 ère adminis-tration | 24 heures après la 1 ère adminis-tration |
| 1 | 10 | - 30% | - 41% | - 34% | - 25% |

5) <u>Etude de la toxicité aigüe</u>

On évalue les doses létales 50 (DL 50) des produits après administration par voie intrapéritonéale chez la souris.

La mortalité est relevée quarante-huit heures après l'administration des produits.

La dose létale 50 (DL 50) du produit décrit à l'exemple 1 est égale à 250 mg/kg ; celle du produit décrit à l'exemple 3 est égale à 40 mg/kg.

6) <u>Détermination de l'activité antidépressive</u>

a) <u>Hypothermie à la réserpine</u>

Le test est effectué sur un lot de 10 souris mâles auxquelles on administre par voie intrapéritonéale 4 mg/kg de réserpine.

La température rectale des animaux est mesurée pendant 5 heures à l'aide d'un thermomètre électrique.

A la dose de 10 mg/kg I.P, le produit de l'exemple 1 retarde l'apparition de l'hypothermie provoquée par la réserpine.

b) <u>Hypothermie à l'apomorphine</u>

Le test est effectué sur un lot de 10 souris mâles.

On induit chez les animaux une hypothermie par injection intrapéritonéale de 16 mg/kg de chlorhydrate d'apomorphine. Le composé testé est administré par voie intrapéritonéale 30 mm avant l'injection d'apomorphine ; la température rectale des animaux est mesurée 30 mm après l'injection d'apomorphine.

Des animaux témoins reçoivent soit du soluté physiologique, soit de l'apomorphine ; on mesure l'écart de température entre les témoins ayant reçu le soluté physiologique, les témoins ayant reçu l'apomorphine seule et les animaux ayant reçu l'apomorphine et le composé testé.

On appelle DE 50 la dose de composé qui réduit de 50 % l'écart de température observé entre les témoins.

La DE 50 du produit de l'exemple 1 a été trouvée égale à 25 mg/kg.

Revendications :

1) Nouveaux dérivés de 1'α-phényl 2-pyrrolidineméthanol ainsi que leurs sels d'addition avec les acides minéraux ou organiques, caractérisés en ce qu'ils répondent à la formule générale (I) :

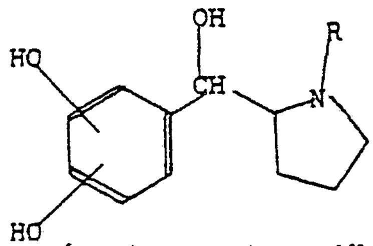

(I)

dans laquelle R représente un atome d'hydrogène ou un radical alcoyle renfermant de 1 à 5 atomes de carbone sous toutes leurs formes stéréoisomères isolées ou en mélange, racémiques ou optiquement actives.

2) Nouveaux dérivés de 1'α -phényl 2-pyrrolidineméthanol, selon la revendication 1 ainsi que leurs sels, caractérisés en ce que dans la formule (I) de la revendication 1, les groupements hydroxyles sont en positions 3 et 4 ou 3 et 5 et R représente un atome d'hydrogène.

3) Le 4-/α-hydroxy (pyrrolidin-2-yl)méthyl/1,2-benzène diol sous ses formes optiquement actives ou racémique ainsi que ses sels.

4) L'oxalate acide de l'isomère A du 4-/α-hydroxy (pyrrolidin-2-yl)méthyl/1,2-benzène diol sous ses formes optiquement actives ou racémique.

5) Procédé de préparation des dérivés de formule (I) tels que définis à la revendication 1 ainsi que de leurs sels, caractérisé en ce que l'on fait réagir le bromure de 4-bromobutyl triphénylphosphonium avec un aldéhyde de formule (II) :

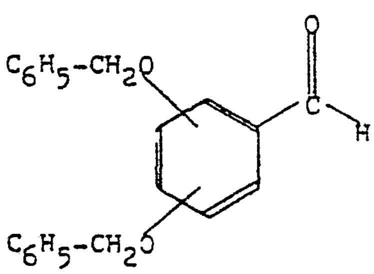

(II)

pour obtenir un produit de formule (III) :

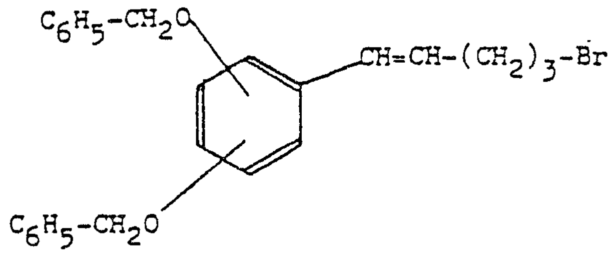

(III)

que l'on fait réagir avec une amine de formule (IV) :

$$R' - NH_2 \qquad (IV)$$

dans laquelle R' représente un radical benzyle ou un radical alcoyle renfermant de 1 à 5 atomes de carbone, pour obtenir un produit de formule (V) :

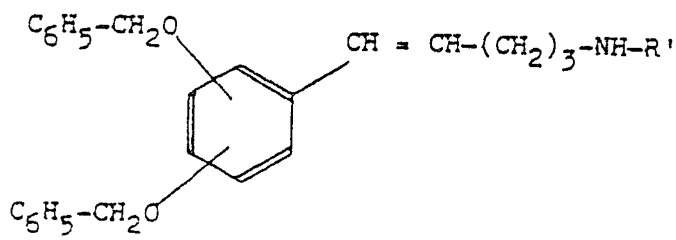

(V)

dans laquelle R' a la signification déjà indiquée, produit de formule (V) que l'on traite par l'anhydride trifluoroacétique, pour obtenir un produit de formule (VI) :

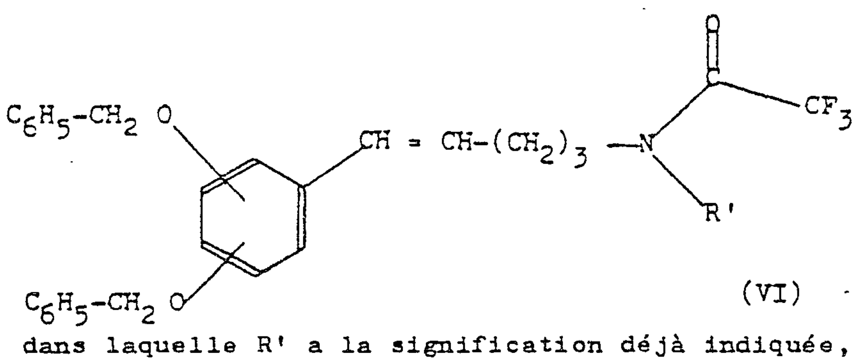

(VI)

dans laquelle R' a la signification déjà indiquée, produit de formule (VI) que l'on traite par le N-bromo succinimide, pour obtenir un produit de formule (VII) :

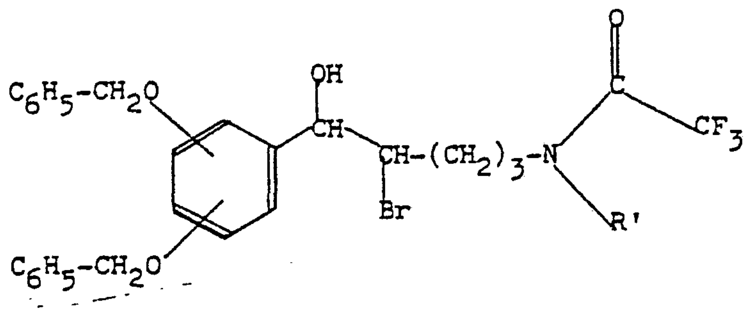

(VII)

dans laquelle R' a la signification déjà indiquée, produit de formule (VII) que l'on traite par un agent alcalin, pour obtenir un produit de formule (VIII) :

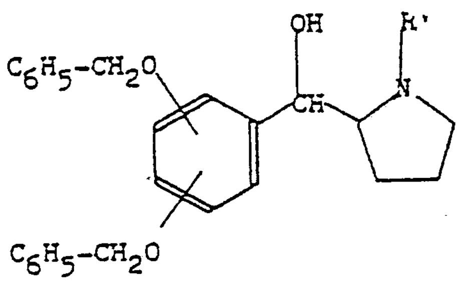

(VIII)

dans laquelle R' a la signification déjà indiquée, sous forme d'un mélange d'isomères A ($VIII_A$) et B ($VIII_B$) que l'on sépare si désiré, produit de formule (VIII), ($VIII_A$) ou ($VIII_B$) que l'on soumet à une hydrogénolyse, pour obtenir le produit de formule (I) recherché sous forme d'un mélange d'isomères A et B que l'on sépare, si désiré, ou sous forme d'isomère A ou B, produit de formule (I) que, si désiré,

- soit l'on salifie,
- soit lorsque R représente un atome d'hydrogène, l'on fait réagir avec un halogénure de formule (IX) :

Hal-R" (IX)

dans laquelle Hal représente un atome de chlore, de brome ou d'iode et R" représente un radical alcoyle renfermant de 1 à 5 atomes de carbone, pour obtenir un produit de formule (I') :

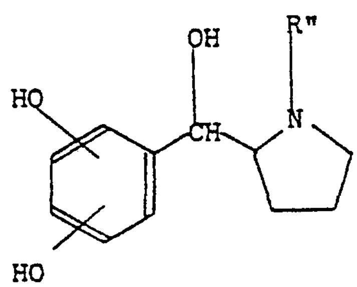

(I')

dans laquelle R" a la signification déjà indiquée correspondant à un produit de formule (I) dans laquelle R a la définition de R" sous forme d'un mélange d'isomères A et B que, si désiré, l'on sépare, ou sous forme d'isomère A ou B, produit de formule (I') que, si désiré, l'on salifie.

6) Procédé de préparation des dérivés de formule (I), tels que définis à la revendication 1 ainsi que de leurs sels, formule I dans laquelle les hydroxyles sont en position 2,4 ou 2,5 ou 3,4 , caractérisés en ce que l'on traite à l'aide de brome un produit de formule X :

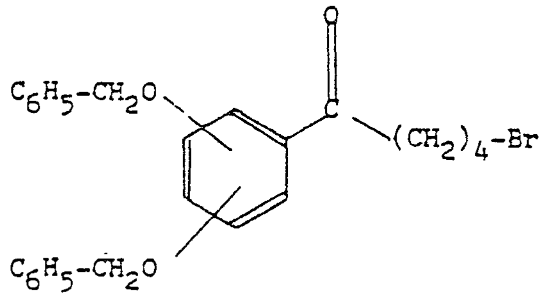

(X)

dans laquelle les radicaux benzyloxy sont en position 2,4 ou 2,5 ou 3,4 , pour obtenir un produit de formule ( XI) :

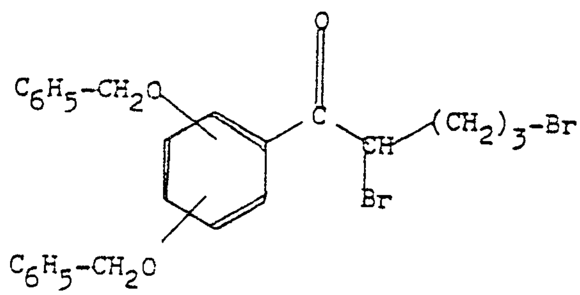

(XI)

que l'on cyclise à l'aide d'une amine de formule (IV) :

$$R' - NH_2 \qquad (IV)$$

que laquelle R' représente un radical benzyle ou un radical alcoyle renfermant de 1 à 5 atomes de carbone pour obtenir un produit de formule (XII) :

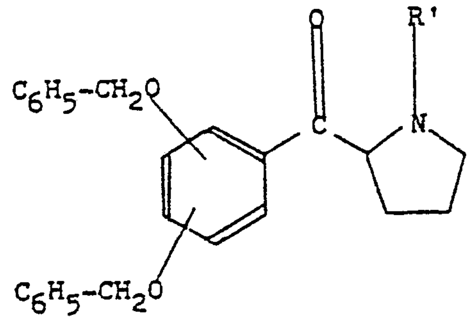

(XII)

dans laquelle R' a la signification déjà indiquée, produit de formule (XII) que l'on réduit, pour obtenir un produit de formule (XIII) :

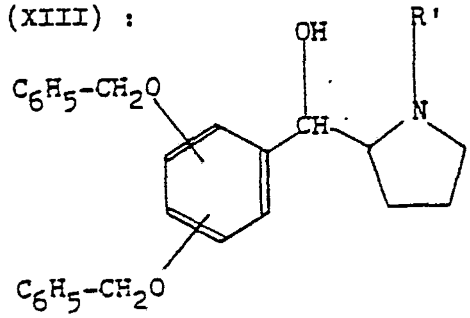

(XIII)

dans laquelle R' a la signification déjà indiquée, sous forme d'un mélange d'isomères A ($XIII_A$) et B ($XIII_B$) que l'on sépare, si désiré, produit de formule (XIII), ($XIII_A$) ou ($XIII_B$) que l'on soumet à une hydrogénolyse, pour obtenir le produit de formule (I) recherché sous forme d'un mélange d'isomères A et B que l'on sépare, si désiré, ou sous forme d'isomère A ou B, produit de formule (I) que, si désiré,

- soit l'on salifie,
- soit lorsque R représente un atome d'hydrogène, l'on fait réagir avec un halogénure de formule (IX) :

Hal-R" (IX)

dans laquelle Hal et R" ont la signification déjà indiquée, pour obtenir un produit de formule (I") :

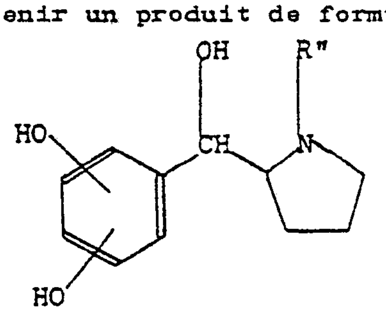

(I")

dans laquelle R" a la signification déjà indiquée, correspondant à un produit de formule (I) dans laquelle R a la définition de R" et les substituants OH sont en position 2,4 ou 2,5 ou 3,4 , sous forme d'un mélange d'isomères A et B que, si désiré, l'on sépare, ou sous forme d'isomère A ou B, produit de formule (I") que, si désiré, l'on salifie.

7) Procédé de préparation selon la revendication 6, caractérisé en ce que le produit de formule (XII) est réduit à l'aide de borohydrure de sodium dans le méthanol, selon que l'on veut de préférence obtenir l'isomère A du produit de formule (I) cherché, ou à l'aide de dihydrure de diéthylsodiumaluminium dans le toluène, selon que l'on veut de préférence obtenir l'isomère B du produit de formule (I) cherché.

8) Procédé de préparation des dérivés de formule (I) tels que définis à la revendication 1, sous la forme de l'isomère A, ainsi que de leurs sels, procédé qui consiste à traiter un produit de formule (X) :

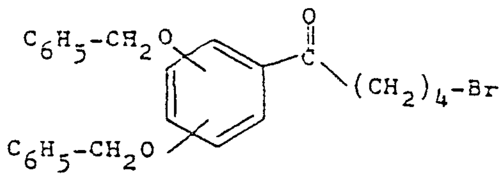

(X)

par le brome, pour obtenir un produit de formule (XI) :

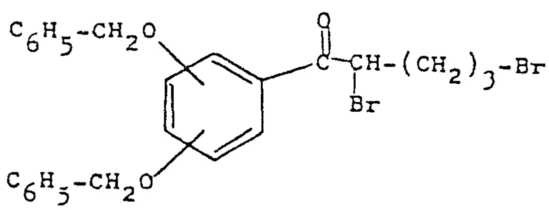

(XI)

et qui est caractérisé en ce que l'on traite ledit produit de formule XI par un agent réducteur, pour obtenir un produit de formule (XIV) :

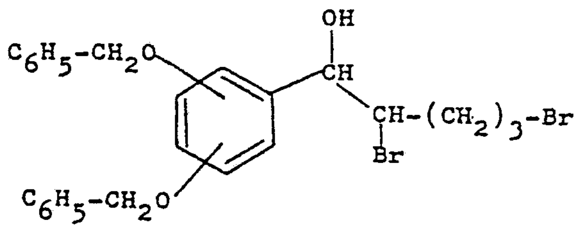 (XIV)

que l'on traite par un alcoolate alcalin, pour obtenir un produit de formule (XV) :

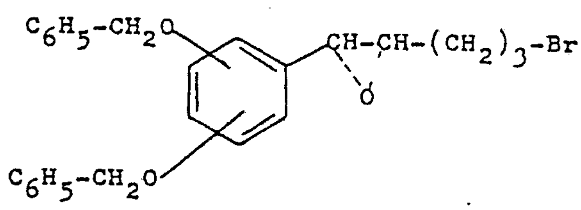 (XV)

que l'on traite par une amine de formule (IV) :

$R'-NH_2$ (IV)

dans laquelle R' représente un radical benzyle ou un radical alcoyle renfermant de 1 à 5 atomes de carbone, pour obtenir un produit de formule (XIII) telle que définie à la revendication 6, sous forme d'isomère A, produit que, l'on soumet à une hydrogénolyse, pour obtenir le produit de formule (I) correspondant, sous forme d'isomère A, produit que, si désiré,
- soit l'on salifie,
- soit lorsque R représente un atome d'hydrogène, l'on fait réagir avec un halogénure de formule (IX) :

Hal - R" (IX)

dans laquelle Hal et R" ont la signification déjà indiquée, pour obtenir un produit de formule (I"') :

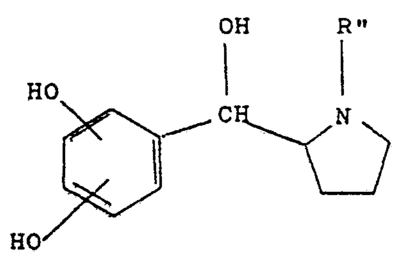 (I"')

dans laquelle R" a la signification déjà indiquée, correspondant à un produit de formule I dans laquelle R a la définition de R", sous forme d'isomère A, produit de formule (I''') que, si désiré, l'on salifie.

9) Procédé selon la revendication 8, caractérisé en ce que l'époxydation est effectuée par le teramylate de sodium au sein du tétrahydrofuranne.

10) Procédé de préparation des dérivés de formule (I), tels que définis à la revendication 1, sous la forme de l'isomère A, ainsi que de leurs sels, procédé qui consiste à faire réagir le bromure de 4-bromobutyl triphényl phosphonium avec un aldéhyde de formule (II) :

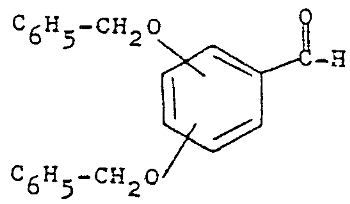

(II)

pour obtenir un produit de formule (III) :

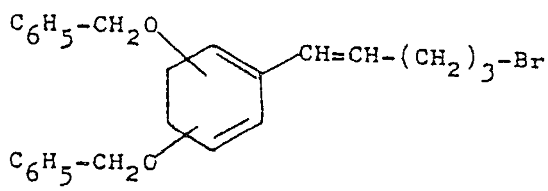

(III)

sous forme de l'isomère cis, et qui est caractérisé en ce que l'on traite ledit produit de formule III par un agent d'époxydation, pour obtenir un produit de formule (XV) telle que définie à la revendication 8 et poursuit la synthèse comme décrit à la revendication 8, à partir de ce produit de formule XV.

11) Médicaments, caractérisés en ce qu'ils sont constitués par les nouveaux dérivés de l'α- phényl 2-pyrrolidineméthanol, tels que définis par la formule (I) de la revendication 1 ainsi que par leurs sels d'addition avec les acides pharmaceutiquement acceptables.

12) Médicaments selon la revendication 11, caractérisés en ce qu'ils sont constitués par les nouveaux dérivés de l'α-phényl

2-pyrrolidineméthanol, tels que définis dans l'une des revendications 2 ou 3, ainsi que par leurs sels d'addition avec les acides pharmaceutiquement acceptables.

13) Médicaments selon la revendication 11 ou 12, caractérisés en ce qu'ils sont constitués par les dérivés de l' α-phényl 2-pyrrolidineméthanol tels que définis dans la revendication 4.

14) Compositions pharmaceutiques, caractérisées en ce qu'elles renferment à titre de principe actif, l'un au moins des médicaments, tels que définis à l'une des revendications 11, 12 ou 13.

0022408

Office européen des brevets

# RAPPORT DE RECHERCHE EUROPEENNE

Numero de la demande

EP 80 40 1004

| DOCUMENTS CONSIDERES COMME PERTINENTS | | | CLASSEMENT DE LA DEMANDE (Int Cl. 3) |
|---|---|---|---|
| Catégorie | Citation du document avec indication, en cas de besoin, des parties pertinentes | Revendication concernee | |
| | FR - A - 2 410 649 (BYK GULDEN LOMBERG)<br>* Pages 1-22, 33-36 *<br>---- | 1,5-9 | C 07 D 207/08//<br>C 07 C 103/38 |
| | | | DOMAINES TECHNIQUES RECHERCHES (Int Cl. 3) |
| | | | C 07 D 207/08 |
| | | | CATEGORIE DES DOCUMENTS CITES<br>X particulièrement pertinent<br>A arrière-plan technologique<br>O divulgation non-écrite<br>P document intercalaire<br>T théorie ou principe à la base de l'invention<br>E demande faisant interférence<br>D document cité dans la demande<br>L document cité pour d'autres raisons |
| | | | & membre de la même famille, document correspondant |
| X | Le présent rapport de recherche a été établi pour toutes les revendications | | |

| Lieu de la recherche | Date d'achèvement de la recherche | Examinateur |
|---|---|---|
| La Haye | 07-10-1980 | FRANCOIS |

OEB Form 1503.1 06.78